# EUROPEAN PATENT APPLICATION

(11) **EP 4 464 265 A1**
(43) Date of publication of application: **20.11.2024**
(21) Application number: 24168779.7
(22) Date of filing: 05.04.2024
(51) Int. Cl.: A61B 17/17, A61B 90/00, A61F 2/46

(54) **PELVIC ORIENTATION VERIFICATION DEVICE**

(30) Priority: 05.04.2023 NL 2034506
(71) Applicant: Massmedica S.A., 02-972 Warschau (PL); Orpidem B.V., 6671 DC Zetten (NL)
(72) Inventor: VERMAAT, Jan, 6671DC Zetten (NL)
(74) Representative: Verhees, Godefridus Josephus Maria

(57) **Abstract**

The verification device 1 has a circular bubble spirit level compass combination 3 that is mounted on a spirit level carrier 5 that is attached to an upper end of an elongated connecting element 7. At the lower end the connecting element 7 is attached to the cup 9a of a ball joint 9. The other part of the ball joint 9 being a ball 9b rotatable in the cup 9a. The ball 9b is attached to a coupling element 11 that can be coupled to the head of a screw 13. The verification device 1 is provided with fixing means 15 for fixing the ball joint parts 9a and 9b relative to each other. The coupling element 11 comprises a securing slide 17 that locks the head of the screw 13.

## Description

### Technical field of the invention

The invention relates to a pelvic orientation verification device for determining a possible change in the orientation of a patient's pelvis during a surgical procedure. Before preparing an acetabulum for insertion of an acetabular cup the hip joint should be exposed and dislocated in a routine manner. Exposing and dislocating the hip joint can result in a change in the orientation of the pelvis during this surgical procedure. Therefore, the orientation of the pelvis should be registered prior to any manipulation that could alter the orientation of the pelvis, and then compared with the orientation of the pelvis after exposing and preparing the acetabulum and before reaming and/or acetabular cup implantation. In case a difference is noticed between the pelvic position at the start of the procedure and after exposure and dislocation, it allows the surgeon to adjust the orientation of the pelvis. Alternatively it may be decided to adjust the pre-planned acetabular version and/or inclination angle according to the orientation findings.

### Background of the invention

When preparing for and/or applying an acetabular cup into a patient's acetabulum, it is essential that the actual orientation of the pelvis is equal to the orientation prior to any manipulation that could have altered the original orientation. If unnoticed, any difference in the pelvic orientation can strongly influence the functional clinical outcome as it may result in that the version and/or inclination angle of the cup component differ from the angles as pre-planned. A pelvic orientation verification device according to the preamble of claim 1 is known from EP 2 996 639 A2.

### Summary of the invention

An object of the present invention is to provide a pelvic orientation verification device with which the orientation of the pelvis can be established more easily. To this end the pelvic orientation verification device according to the invention is characterized in that the spirit level is a circular bubble spirit level compass combination so that the orientation of the pelvic can be verified in three directions.

With this pelvic orientation verification device the position of the pelvis of a patient can be recorded before and after the hip joint is exposed and dislocated. So, with the device according to the invention a quick and reliable control of the orientation and possible displacement of the pelvis can be obtained, minimising the guesswork for acetabular cup placement.

It may not always be possible to optimally adjust the orientation of the pelvis, in which case the verification device informs the surgeon about the possible need for adjusting the version and/or inclination angle of an acetabular cup reamer/insertion/aiming device. Due to the presence of the compass the value of the necessary adjustment angle in the horizontal plane can be read.

Before any surgical preparation of the acetabulum for insertion of the acetabular cup the anchor screw should be inserted at an appropriate location on the pelvis. Then the connecting element must be connected to the head of the screw, which fits one way only. The ball joint should now be adjusted such that the bubble of the spirit level is in the middle and the position of needle should superimpose a marking present on the compass. Hereafter the ball joint is locked to secure this position. Then the connecting element together with the circular bubble spirit level compass combination must be removed.

Now the hip joint can be exposed and dislocated in the routine manner. Prior to acetabular reaming and/or to cup positioning the orientation of the pelvis should be checked for any orientation change by re-arranging the connecting element and the bubble spirit level compass combination onto the anchor screw. In case a difference between the original orientation and the actual orientation is noticed, the orientation of the pelvis can be adjusted if needed such that the same orientation is reached as before the exposure and dislocation of the hip joint. In the event the orientation of the pelvis cannot be optimally adjusted it may be decided to adjust the pre-planned version and/or inclination angle according the findings.

Now the connecting element and the circular bubble spirit level compass combination should be removed and the acetabulum can be prepared. After preparation and prior to insertion of the acetabular component the procedure, as earlier described, to check the orientation of the pelvis can be repeated. Finally the acetabular cup can be positioned and arranged. The pelvic orientation verification device can be applied with a patient operated in the lateral or supine position.

From US 9 114 027 B2 a pelvic orientation verification device is known in which the spirit level is a bubble spirit level that has no compass features. To orientate an object in 3D space with the aid of this device is more difficult than with the device according to the invention.

The pelvic orientation verification device preferably comprises a spirit level carrier on which the spirit level is mounted and which is connected to the first ball joint parts so that the spirit level can easily be disconnected.

The pelvic orientation verification device further preferably comprises an elongated connecting element which is attached with an end to the first ball joint part and with another end to the spirit level carrier.

If the orientation of the pelvis has to be determined when a patient is lying on its side, the spirit level cannot be positioned in the horizontal plane with the above-described embodiment of the device. In order to also be able to use the device with patients lying on the side, an embodiment of the device is characterized in that the elongated connecting element comprises two parts that are connected to each other via a further joint configured to rotate these parts around an axis perpendicular to these parts, which joint being provided with further fixing means to fix both parts relative to each other.

### Brief description of the drawings

The invention will be further elucidated below on the basis of drawings. These drawings show embodiments of the pelvic orientation verification device according to the present invention. In the drawings:
Figure 1 shows a first embodiment of the pelvic orientation verification device;
Figure 2 shows the tool for screwing the anchor screw into bone;
Figure 3 shows the anchor screw;
Figure 4 shows the bubble spirit level;
Figure 5 shows the connecting element;
Figure 6 shows the assembled pelvic orientation verification device arranged on a pelvis from which the orientation should be determined; and
Figure 7 shows a second embodiment of the pelvic orientation verification device.

### Detailed description of the drawings

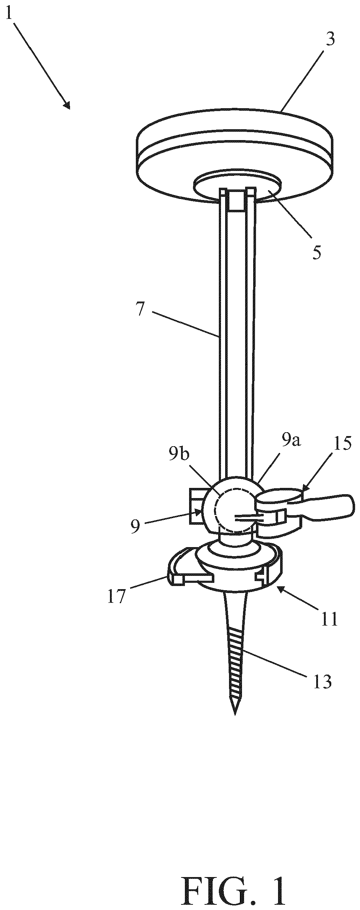

In figure 1 a first embodiment of the pelvic orientation verification device according to the invention is shown. The verification device 1 has a circular bubble spirit level compass combination 3 that is mounted on a spirit level carrier 5 that is attached to an upper end of an elongated connecting element 7. At the lower end the connecting element 7 is attached to the cup 9a of a ball joint 9. The other part of the ball joint 9 being a ball 9b rotatable in the cup 9a. The ball 9b is attached to a coupling element 11 that can be coupled to the head of a screw 13. This screw can be cannulated.

The verification device 1 is provided with fixing means 15 for fixing the ball joint parts 9a and 9b relative to each other. The coupling element 11 comprises a securing slide 17 that locks onto the head of the screw 13.

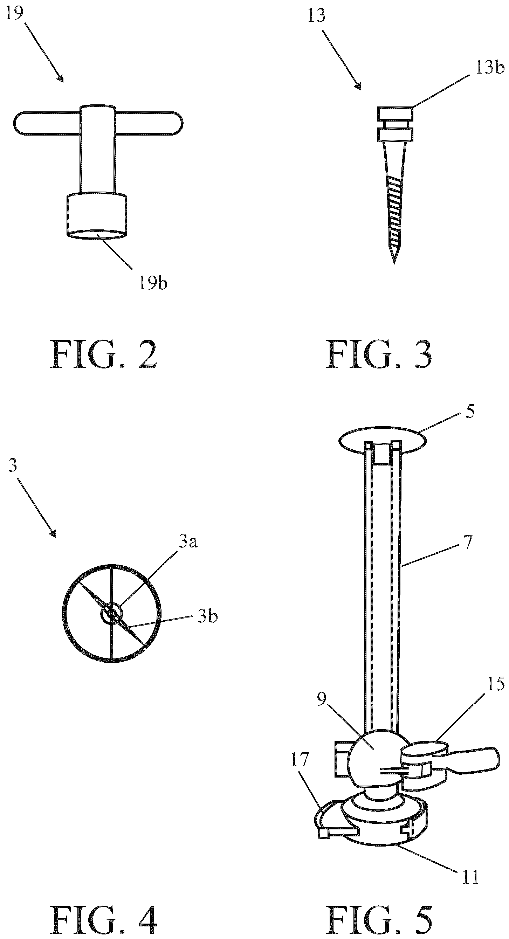

Figure 2 shows a tool 19 for screwing the screw 13 into the pelvis. The tool 19 can be coupled to the head 13b of the screw 13 (figure 3). The head 13b of the screw 13 and the recess 19b in the tool 19 are shaped such that the tool and the head are blocked relative to each other in rotational direction.

Figure 3 shows the circular bubble spirit level compass combination 3. The spirit level compass combination is filled with liquid in which a gas bubble 3a is present. The spirit level compass combination 3 further has a free rotatable needle 3b present in the liquid. The spirit level compass combination 3 further has marking lines for noting the orientation of the needle 3b. Figure 5 shows the verification device without the spirit level compass combination 3 and screw 13.

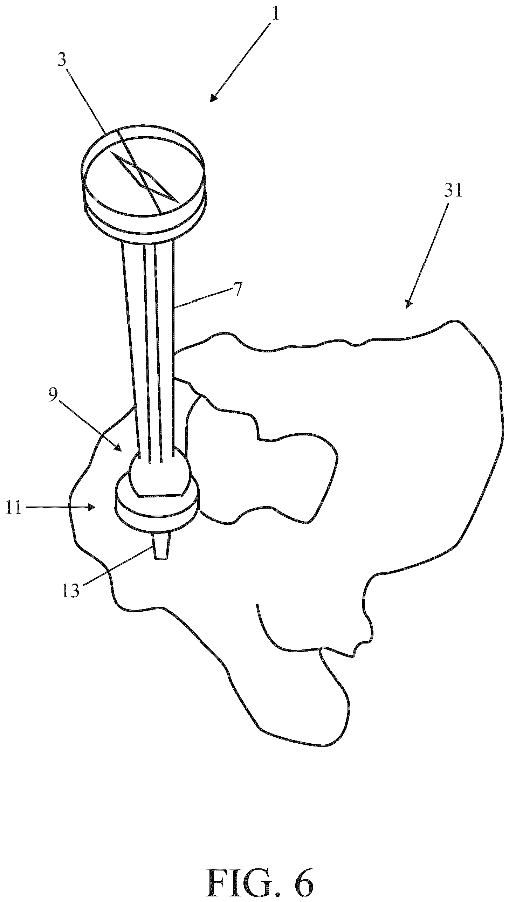

Figure 6 shows the pelvic orientation verification device 1 arranged on a pelvis 31 of which the orientation should be determined. The ball joint 9 and coupling means 11 are schematically shown and not in detail. Before preparing the acetabulum for insertion of the acetabular cup the hip joint should be exposed and dislocated in a routine manner. Exposing, dislocating and surgical manipulation of the hip joint can result in a change in the original orientation of the pelvis. Therefore, the orientation of the pelvis should be checked prior to acetabular reaming and/or acetabular cup insertion and the orientation of the pelvis should be adjusted when necessary to get the same orientation as before any surgical manipulation that may have altered the position of the pelvis.

In order to determine the orientation of the patient's pelvis, first the screw 13 should be screwed into the pelvis 31 with the aid of a dedicated tool 19. Then the connecting element 7 with the spirit level compass combination 3 mounted on it, must be connected to the head of the screw 13. The spirit level compass combination should now be adjusted such that the bubble of the spirit level compass combination is in the middle and the position of the needle of the compass is superimposing the marking line, after which the ball joint is locked in position. Then the connecting element 7 together with the circular bubble spirit level compass combination 3 must be removed. Now the hip joint can be exposed, dislocated and prepared in the routine manner. Then, prior to acetabular reaming and/or cup positioning, the orientation of the pelvis should be checked for any change by re-arranging the connecting element and the bubble spirit level compass combination on the screw. Then, if necessary, the orientation of the pelvis should be adjusted such that the same orientation is reached as before the exposure, dislocation and surgical manipulation of the hip joint. Now the verification device should be removed entirely and the acetabulum can be prepared and the acetabular cup can be positioned and arranged.

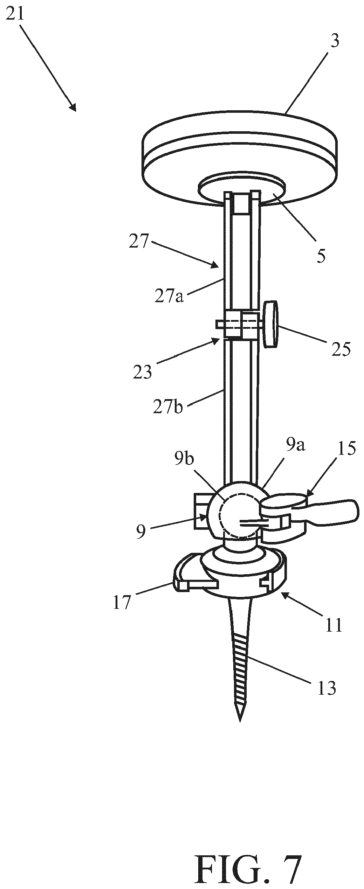

In figure 7 a second embodiment of the pelvic orientation verification device according to the invention is shown. All parts identical to those of the first embodiment described above are designated by the same reference numerals. The elongated connecting element 27 of this device 21 comprises two parts 27a and 27b that are connected to each other via a joint 23. This joint 23 is configured to rotate the parts 27a and 27b around an axis perpendicular to these parts. This joint 23 is also provided with fixing means 25 to fix both parts relative to each other. This embodiment can be used for patients lying on his/her side to record the orientation of the pelvis. With the above described first embodiment it is not possible to position the spirit level in a horizontal plane in case the patient is lying on its side. The additional joint 23 of this device 21 eliminates this shortcoming and makes it possible to position the spirit level in the horizontal plane.

Although the present invention is elucidated above on the basis of the given drawings, it should be noted that this invention is not limited whatsoever to the embodiments shown in the drawings. The invention also extends to all embodiments deviating from the embodiments shown in the drawings within the context defined by the claims.

## Claims

1. Pelvic orientation verification device (1; 21) for determining a possible change in the orientation of a patient's pelvis (31) during a surgical hip procedure and while positioned on an operating table, comprising:
- a ball joint (9) consisting of a first and a second ball joint part formed by a cup (9a) and a ball (9b) that can be turned therein,
- fixing means (15) for fixing the ball joint parts (9a, 9b) relative to each other, and
- a spirit level (3) connected to the first ball joint part (9a),
- an anchor screw (13), for attaching to the patient's pelvis (31), which anchor screw (13) is provided with a head (13b), and
- coupling means (11) for coupling to the head (13b) of the anchor screw (13), which coupling means (11) are attached to the second ball joint part (9b).
**characterized in that** the spirit level (3) is a circular bubble spirit level compass combination.

2. Pelvic orientation verification device (1; 27) as claimed in claim 1, **characterized in that** it further comprises a spirit level carrier (5) on which the spirit level (3) is mounted and which is connected to the first ball joint part.

3. Pelvic orientation verification device (1; 27) as claimed in claim 2, **characterized in that** it further comprises an elongated connecting element (7; 27) which is attached with an end to the first ball joint part (9a) and with another end to the spirit level carrier (5).

4. Pelvic orientation verification device as claimed in claim 3, **characterized in that** the elongated connecting element (27) comprises two parts (27a, 27b) that are connected to each other via a further joint (23) configured to rotate these parts around an axis perpendicular to these parts (27a, 27b), which joint (23) being provided with further fixing means (25) to fix both parts (27a, 27b) relative to each other.
